# EUROPÄISCHE PATENTSCHRIFT

(11) **EP 0 963 738 B1**
(45) Veröffentlichungstag und Bekanntmachung des Hinweises auf die Patenterteilung: **22.09.2004**
(21) Anmeldenummer: 98250199.1
(22) Anmeldetag: 08.06.1998
(51) Int. Cl.: A61C 8/00

(54) **Implantat mit Halterung**
Implant with handle
Implant avec dispositif de maintien

(43) Veröffentlichungstag der Anmeldung: 15.12.1999
(73) Patentinhaber: Ihde, Stefan, Dr., 80802 München (DE)
(72) Erfinder: Ihde, Stefan, Dr., CH-8738 Uetliburg (CH)
(74) Vertreter: Radwer, Dieter

(56) Entgegenhaltungen:
- FR-A- 2 347 027
- US-A- 3 675 328
- US-A- 3 861 043
- US-A- 4 713 004
- US-A- 4 923 471
- US-A- 5 368 160
- US-A- 5 558 230

## Beschreibung

Die Erfindung betrifft ein enossales Dentalimplantat mit einer geeigneten Transportverpackung.

Dentalimplantate müssen gemäß den gültigen Richtlinien so verpackt sein, dass sie während des Transports nicht mit anderen Stoffen oder anderen Metallen als dem Implantatmetall in Berührung kommen. Dieser Vorschrift wird nach dem Stand der Technik so entsprochen, dass die Implantate in Behältern aus Titan (auch die Implantate selber werden aus Titan hergestellt) eingeschlossen werden und mittels Schraubverbindungen auf der der späteren Knochenkavität abgewandeten Seite in diesen Transportbehältern kontaktfrei positioniert bzw. gehaltert sind. Eine Verpackung dieser Art ist beispielsweise aus US 5 558 230 und US 5 368 160 bekannt.

Nach US 5 558 230 ist das Implantat, zusammen mit einer Einheilschraube, unter Ausnutzung des auf dem Außenmantel des Implantates vorgesehenen Gewindes mit seiner basalen Seite in den Verschluss der Verpackung, die das Implantat kontaktfrei umschließt, eingeschraubt. Für die Aufnahmen des Implantates und der Einheilschraube besitzt der Verschluss der Verpackung eine zapfenartige Verlängerung, in die eine Aussparung für die Positionierung der Einheilschraube und stirnseitig eine Gewindebohrung vorgesehen sind, in die das Implantat eingeschraubt wird.

Bei der sterilen Verpackung für Dentalimplantate nach US 5 368 160 wird das Implantat zur kontaktfreien Positionierung mit seiner crestalen Seite unter Verwendung der Einheilschraube in ein Zwischenteil eingeschraubt und über das Zwischenteil, welches bei der Insertion des Implantates als Schrauber dient, in eine entsprechende Aussparung des Verpackungsverschlusses eingesetzt.

Es ist ferner bereits bekannt, dass Dentalstifte, die für den Aufbau der Zahnkrone in die vorhandene Zahnwurzel eingeschraubt werden, oder chirurgische Hilfsmittel zur Behandlung von Knochenbrüchen mit Sollbruchstellen ausgestattet sind, um die nicht benötigten Teile dieser Elemente von den eingeschraubten bzw. eingesetzten Teilen auf einfache Weise zu trennen - US 3 675 328, US 3 861 043, US 4 923 471, FR 2 347 027.

Der vorliegenden Erfindung liegt die Aufgabe zugrunde, ein Dentalimpantat nebst Primärverpackung zu schaffen, welches einfach und billig herzustellen ist und ohne zusätzliche Hilfsteile, insbesondere ohne separate Schrauben und Behältnisse aus Titan, verpackt werden kann.

Erfindungsgemäße wird die Aufgabe durch ein Dentalimplantat und eine (Primär)Verpackung nach den Merkmalen von Anspruch 1 gelöst.

Um ein Implantat auf einfache und kostengünstige Weise sicher zu positionieren, ist nach der vorgeschlagenen Lösung an der basalen Seite des Implantates eine Halterung, die vorteilhafter Weise einstückig mit dem Implantat hergestellt wird, vorgesehen, die das Implantat in Verpackungsstellung im Griffstück der Primärverpackung fixiert, wobei zur Insertion das Implantat und die Halterung in an sich bekannter Weise durch die Sollbruchstelle, die im Fußbereich des Implantates vorgesehen ist, voneinander trennbar sind.

Weitere vorteilhafte Ausbildungen der Erfindung ergeben sich aus den Unteransprüchen 2 bis 5.

Da die nach dem Einbringen des Implantats dem Mund zugewandte Seite des Implantats regelmäßig mit präzise hergestellten Verbindungsflächen oder Gewinden versehen ist, kann in diesen Bereichen keine zusätzliche einstückige Halterung angebracht werden. Die Anbringung einer einstückigen Halterung auf der Knochenseite wurde bislang nicht erwogen, da nach dem Trennen der Verbindung von Halterung und Implantat im Bereich der erfindungsgemäßen Sollbruchstelle keine Passivierung der Oberflächen vorliegen könne. Dies würde per se zu einer schlechteren Einheilung des Implantats führen. Es zeigt sich jedoch klinisch, dass diese Annahme nicht stimmt und dass innerhalb von wenigen Sekundenbruchteilen nach dem Trennen von Implantat und Halterung im Bereich der Bruchstelle eine vergleichbare Passivierung vorliegt wie in anderen Bereichen des Implantats.

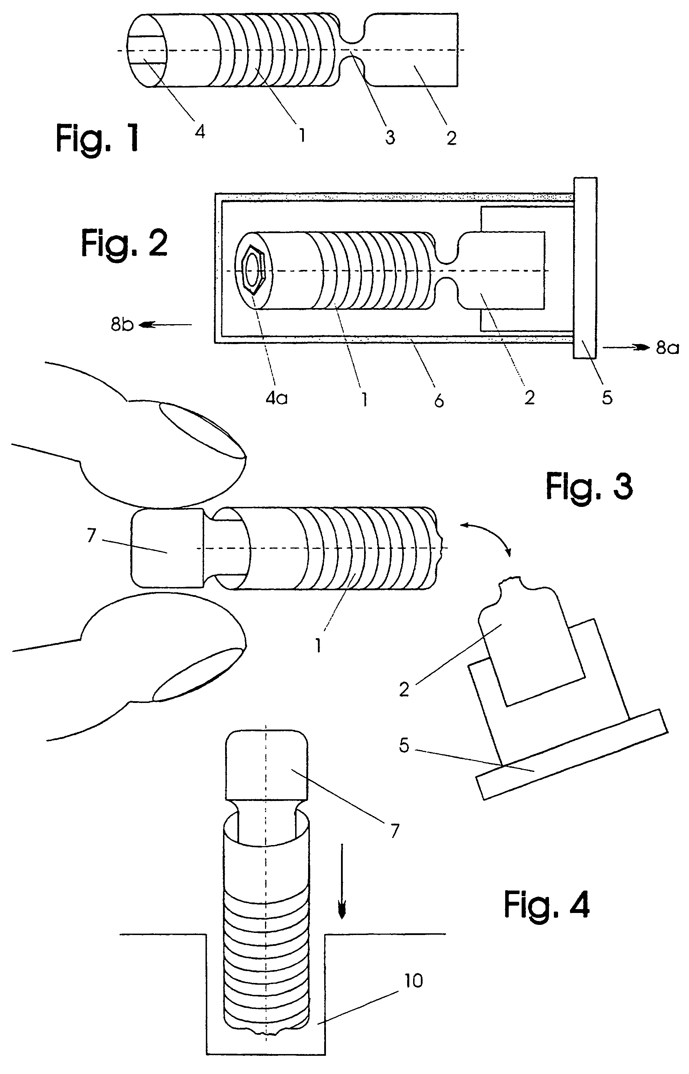

Die vom Implantat abgetrennte Halterung kann auch als eigentlicher Griff verwendet werden, wodurch die Verwendung eines Einbring-Hilfsteiles, wie in Fig. 4 dargestellt, entfällt.

Die Erfindung soll nachstehend an einem Ausführungsbeispiel näher erläutert werden. In der dazugehörigen Zeichnung zeigen:
- Fig. 1: eine schematische Darstellung des erfindungsgemäßen Implantates mit angeformter Halterung,
- Fig. 2: das nach der Erfindung in eine sterile Verpackung eingesteckte und kontaktfrei positionierter Implantat,
- Fig.3: den Trennvorgang von Implantat und Halterung vor der Insertion des Implantates unter Verwendung eines Einbring-Hilfsteiles,
- Fig. 4: die Insertion des Implantates in ein Implantatbett in schematischer Darstellung.

Wie aus Fig. 1 ersichtlich, wird das erfindungsgemäße Implantat durch ein Implantat mit einem enossalen Teil 1 und einer Halterung 2 gebildet wobei Implantat 1 und Halterung 2 einstückig hergestellt sind und über eine Sollbruchstelle 3, die im Fußbereich des Implantates 1 vorgesehen ist, miteinander verbunden sind. Die dem Mund zugewandte Seite des Implantats 1 ist in der dargestellten Ausführungsform mit einem sog. internen Hexagon 4 ausgestattet. Es kann jedoch auch ein externer Hexagon 4a (Fig. 2) oder jede andere Implantat-Abutment-Verbindung beim erfindungsgemäßen Implantat angebracht oder integriert sein.

Gemäß Fig. 2 ist das in Fig. 1 dargestellte Implantat 1 in einer sterilen Primärverpackung 6 in Form eines Röhrchens untergebracht, die das Implantat allseitig umschließt, wobei die Halterung 2 in ein Griffstück 5 eingesteckt ist, das Teil der Primärverpackung ist und als Verschluss des Röhrchens dient. Mit 8a und 8b sind die Richtungen bezeichnet, in die Röhrchen und Griffstück 5 gegeneinander gezogen werden, um die Packung zu öffnen. Erfindungsgemäß kann die Halterung 2 in das Griffstück 5 einrasten, sie kann eingeschraubt werden oder durch Klebung mit dem Griffstück 5 verbunden sein.

Fig. 3 zeigt, wie nach dem Entfemen des Röhrchens das Implantat 1 mit einem praxisüblichen Einbring-Hilfsteil 7 verbunden und gegen die Halterung 2 abgeknickt wird. Dabei wird die Halterung vorzugsweise am Griff 5 angefasst. Altemativ können Griffstück 5 und Halterung 2 auch einstückig hergestellt sein. In diesem Fall ist die Halterung 2 dann Bestandteil der Primärverpackung 6.

Fig. 4 zeigt das Einbringen des Implantats in die Knochenkavität 10. Dabei wird das Implantat an dem Einbring-Hilfsteil 7 festgehalten.

### BEZUGSZEICHENAUFSTELLUNG

- 1: Implantat/enossaler Teil
- 2: Halterung
- 3: Sollbruchstelle
- 4: internes Hexagon
- 4a: externes Hexagon
- 5: Griffstück
- 6: Primärverpackung
- 7: Einbring-Hilfsteil
- 8a: Öffnungsrichtung
- 8b: Öffnungsrichtung
- 9: -
- 10: Knochenkavität

## Patentansprüche

1. Dentalimplantat mit steriler Verpackung , das zum Lösten von einem Griffstück, welches Teil der Verpackung ist, eine Sollbruchstelle aufweist und an der zur Sollbruchstelle abgewandten Seite mit Mitteln zur Verhinderung einer Verdrehung des Implantataufbaus versehen ist, während die basale Seite des Implantates ein Gewinde zum Einschrauben des Implantates in den Kieferknochen träge, wobei an der basalen Seite des Implantates (1) eine Halterung (2) vorgesehen ist, die das Implantat (1) in Verpackungsstellung im Griffstück (5) der Primärverpackung (6) fixiert, wobei das Implantat (1) und die Halterung (2) in an sich bekannter Weise durch eine Sollbruchstelle (3), die im Fußbereich des Implantates (1) vorgesehen ist, voneinander trennbar sind.

2. Dentalimplantat nach Anspruch 1, **dadurch gekennzeichnet, dass** die Halterung (2) und das Griffstück (5) gegeneinander einrasten.

3. Dentalimplantat nach Anspruch 1, **dadurch gekennzeichnet, dass** die Halterung (2) und das Griffstück (5) miteinander verschraubt sind.

4. Dentalimplantat nach Anspruch 1, **dadurch gekennzeichnet, dass** die Halterung (2) und das Griffstück (5) miteinander verklebt sind.

5. Dentalimplantat nach Anspruch 1, **dadurch gekennzeichnet, dass** Implantat (1), Halterung (2) und Griffstück (5) einstückig hergestellt und in ein Röhrchen der Primärverpackung (6) steril eingesteckt sind.

## Claims

1. Dental implant with sterile packaging, having a defined separation site to release a grip, which is part of the packaging, and which has, at the side turned toward the defined separation site, means to prevent twisting of the implant structure, while the basal side of the implant has a thread for screwing the implant into the jaw bone, whereby a holder (2) which fixes the implant (1) in the grip (5) of the primary package is provided at the basal side of the implant (1) at the packaging position, whereby the implant (1) and the holder (2) can be separated from each other in a way which is itself known by means of a defined separation site (3) provided in the foot region of the implant (1).

2. Dental implant according to Claim 1, **characterized in that** the holder (2) and the grip (5) snap together.

3. Dental implant according to Claim 1, **characterized in that** the holder (2) and the grip (5) are screwed together.

4. Dental implant according to Claim 1, **characterized in that** the holder (2) and the grip (5) are cemented together.

5. Dental implant according to Claim 1, **characterized in that** the implant (1), holder (2) and grip (5) are manufactured in one piece and inserted sterile into a tube in the primary package (6).

## Revendications

1. Implant dentaire avec emballage stérile, présentant une zone de cassure pour se détacher d'une partie de préhension qui fait partie de l'emballage et qui est pourvu sur le côté opposé à la zone de cassure de moyens empêchant la rotation de la structure d'implant, tandis que la partie basale de l'implant porte un filetage pour le vissage de l'implant dans l'os de la mâchoire, dans lequel un dispositif de maintien (2) est prévu sur le côté basal de l'implant (1) et fixe l'implant (1) dans la position d'emballage dans la partie de préhension (5) de l'emballage primaire (6), l'implant (1) et le dispositif de maintien (2) pouvant être séparés l'un de l'autre de manière connue en soi dans une zone de cassure (3) prévue dans la zone de pied de l'implant (1).

2. Implant dentaire selon la revendication 1, **caractérisé en ce que** le dispositif de maintien (2) et la partie de préhension (5) s'emboîtent l'un dans l'autre.

3. Implant dentaire selon la revendication 1, **caractérisé en ce que** le dispositif de maintien (2) et la partie de préhension (5) sont vissés l'un à l'autre.

4. Implant dentaire selon la revendication 1, **caractérisé en ce que** le dispositif de maintien (2) et la partie de préhension (5) sont collés l'un à l'autre.

5. Implant dentaire selon la revendication 1, **caractérisé en ce que** l'implant (1), le dispositif de maintien (2) et la partie de préhension (5) sont fabriqués d'un seul tenant et introduits dans des conditions stériles dans un tube de l'emballage primaire (6).c
